(19) Europäisches Patentamt
European Patent Office
Office européen des brevets

(11) Publication number : **0 552 024 A2**

# (12) EUROPEAN PATENT APPLICATION

(21) Application number : **93300197.6**

(22) Date of filing : **13.01.93**

(51) Int. Cl.$^5$ : **A61K 7/06,** A61K 7/48, A61K 7/42, A61K 9/107

(30) Priority : **15.01.92 GB 9200764**

(43) Date of publication of application : **21.07.93 Bulletin 93/29**

(84) Designated Contracting States : **AT BE CH DE DK ES FR GB GR IE IT LI NL PT SE**

(71) Applicant : **UNILEVER PLC**
**Unilever House Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Birtwistle, David Howard**
**18 Exmoor Close**
**Irby, Wirral L61 92N (GB)**
Inventor : **Parkington, Michael John**
**1 Gerard Road, West Kirby**
**Wirral, Merseyside L48 4ES (GB)**
Inventor : **O'Shea, Gerald Joseph**
**54 Irby Road**
**Heswall, Wirral L61 6XS (GB)**

(74) Representative : **Ford, Michael Frederick et al**
**MEWBURN ELLIS 2 Cursitor Street**
**London EC4A 1BQ (GB)**

## (54) Cosmetic composition with enhanced deposition of cosmetic agents.

(57) A rinse-off cleansing composition including one or more surfactant-soluble cosmetic agents for deposition onto hair or skin, the composition comprising a stable emulsion having a continuous phase comprising one or more surfactants and an internal phase comprising one or more oil materials, wherein the internal oil phase contains the said one or more surfactant-soluble cosmetic agents. The use of the oil phase to dissolve the one or more surfactant-soluble cosmetic agents reduces the degree of solubilization of the cosmetic agent into micelles of surfactant in the surfactant-comprising continuous phase, such that deposition of the cosmetic agent onto a surface such as hair or skin is enhanced. Preferred cosmetic agents are oil sunscreens and preferred oil materials for use in the internal phase include phenylsilicones, mineral oil and organic oils.

EP 0 552 024 A2

The present invention relates to cosmetic compositions, particularly to rinse-off cleansing compositions which include one or more cosmetic agents to be deposited onto the hair or the skin. The invention is especially concerned with enhancing the deposition of cosmetic agents from such compositions, compared with that from similar compositions of this type found in the prior art.

Difficulties frequently arise in achieving effective deposition of cosmetic agents onto hair or skin when the cosmetic agent is delivered by means of incorporation into rinse-off compositions, typically hair and body shampoos. Especially cosmetic agents which are water- and/or surfactant- soluble are often preferentially rinsed away from the intended site of deposition, rather than being deposited thereat and there have hitherto been numerous proposals in the art seeking to enhance deposition of such materials from these kinds of compositions.

This problem is particularly pronounced in the context of shampoos, i.e. cleansing compositions, where the cosmetic agent to be deposited is surfactant-soluble, yet may still be water-insoluble. Hitherto there has been little, even no, study of deposition problems of specifically surfactant-soluble, often oily, cosmetic agents from this type of system.

There is much prior art relating to oil-based cosmetic compositions such as in the form of creams, lotions, gels, milks or the like, e.g. comprising emulsions, which contain oily cosmetic agents such as sunscreens for deposition onto skin. As well as being intended primarily as a leave-on, rub-off-resistant and often water-resistant type of product, these compositions also contain no or little surfactant, so are unsuitable for cleansing purposes, either for the skin or for the hair.

Modification of these known compositions by simple addition of surfactant in order to give desirable detergency properties is not possible, because little deposition of the sunscreen or other cosmetic agent which is surfactant-soluble results, as a consequence of the agent being solubilized by the surfactant and easily carried away from the intended site of deposition as the composition is rinsed off.

There therefore exists in the art a problem of poor deposition of suspended particles of a surfactant-soluble cosmetic agent from a rinse-off cleansing composition containing significant amounts of surfactant. Among other disadvantages, this leads to non-cost-effective use of and waste of (which may possibly be undesirable for environmental or health reasons) cosmetic raw materials.

US 3580853 discloses detergent compositions such as shampoos which comprise anionic surfactant, water-insoluble particles of material and a particular defined cationic polymer as a deposition enhancer for the particulate material.

EP-A-117135 discloses shampoos which contain a water-soluble nitrogen-containing polymer in addition to a water-soluble non-particulate material such as a sunscreen.

EP-A-0093601 discloses washing compositions which comprise anionic surfactant, a water-insoluble particulate substance, e.g. an oily material, and a water-soluble cationic polymer.

EP-A-0419164 discloses rinse-off hair conditioning compositions containing an aqueous phase and a lamellar phase comprising cationic surfactant, and an oil sunscreen is present in the lamellar phase.

EP-A-0468721 discloses shampoo compositions which comprise surfactant, an aqueous emulsion of a solution of highly viscous silicone in volatile solvent and a cationic derivative of guar gum, wherein the average particle size of the highly viscous silicone in the composition is less than 2 microns.

EP-A-0386898 discloses aqueous shampoo compositions comprising, in addition to water, anionic surfactant, a water-insoluble sunscreen material and a cationic polymer as a deposition agent.

Surprisingly, we have now found that it is possible to ameliorate the above problems and to enhance deposition of surfactant-soluble cosmetic agents from rinse-off cleansing compositions, by use of an additional oil phase in the composition. The oil has the effect of reducing solubilization of the cosmetic agent into the phase comprising the surfactant, thereby enhancing deposition of the cosmetic agent when the composition is rinsed off.

Thus, the invention resides in the use in rinse-off shampoos of oils into which surfactant-soluble ingredients would otherwise partition if incorporated directly into the composition. The effect of this is to reduce the amount of cosmetic agent solubilized into micelles of surfactant in the surfactant-comprising phase of the composition, such that deposition of the cosmetic agent onto a surface which is contacted with the compositions is enhanced.

Accordingly, in one aspect the present invention provides a rinse-off cleaning composition including one or more surfactant-soluble cosmetic agents for deposition onto hair or skin, the composition comprising a stable emulsion having a continuous phase comprising one or more surfactants and an internal phase comprising one or more oil materials, wherein the internal oil phase contains the said one or more surfactant-soluble cosmetic agents.

The invention, and in particular preferred aspects, features and embodiments thereof, will now be described in detail.

The cosmetic agent

Compositions in accordance with the invention are advantageous for facilitating deposition of cosmetic agents which are surfactant-soluble and for which difficulties in achieving efficient and cost-effective deposition have hitherto been encountered. By "surfactant-soluble" is meant that the cosmetic agent would otherwise be solubilizable within micelles of the surfactant in the surfactant-containing phase of the composition to a degree such that the agent would be predominantly carried away in the micelles as the composition is rinsed off.

For the purpose of this invention, the cosmetic agent may be any surfactant-soluble material (liquid or solid) intended to be deposited onto the hair or the skin for imparting one or more cosmetic or other desirable benefits, e.g. for hair/skin health or for one or more visual and/or tactile benefits.

Preferred cosmetic agents suitable for use in the invention are hydrophobic materials (i.e. water-insoluble materials), such as cosmetic oil materials, which are soluble in micelles of the surfactant of the cleansing composition.

Examples of such suitable materials include, but are not limited to, sunscreen materials e.g. UV absorbers, after-sun treatment materials, occlusive oils, emollients, humectants, therapeutic and essential oils, perfumes, antiperspirants, moisturisers, colour cosmetic materials, and even combinations of such materials, for example where a combination of cosmetic benefits is desired to be delivered by the composition.

A particularly useful application of the invention is for enhancing deposition of surfactant-soluble sunscreen materials onto hair or skin from cleansing compositions such as shampoos.

Suitable sunscreens include benzophenone compounds, dibenzoyl methane derivatives, camphor derivatives and cinnamate derivatives which have the defined properties required of this invention. Examples of these species of sunscreens are well known in the art and widely commercially available. Specific examples are those disclosed in any of the prior art references mentioned above, which disclosures are incorporated herein by reference. One particularly preferred sunscreen is a UV absorber such as Parsol MCX (2-ethyl hexyl methoxy cinnamate) from GIVAUDAN Co Limited.

The oil material

In the compositions according to the invention the surfactant-soluble cosmetic agent is present in the internal oil phase of the emulsion and is preferably dissolved therein, whereby the internal phase of the emulsion comprises a solution of the cosmetic agent in an oil, which may be a mixture of oil components.

Preferred oil materials for forming the internal oil phase of the emulsion are therefore those which dissolve the one or more cosmetic agents to be deposited. The oil phase should generally permit sufficient of the cosmetic agent(s) to be dissolved therein (or rather the cosmetic agent(s) should be sufficiently soluble therein) so that the required amount of cosmetic agent is able to be deposited from a given quantity of the composition.

Ideally (though there is no express limitation to this), the oil material(s) forming the oil phase is surfactant-insoluble, so that it is not solubilized in micelles of the surfactant in the continuous phase of the composition, but remains as stable suspended droplets or particles containing the cosmetic agent(s). However, this ideal may be difficult to achieve with many practical systems utilising commonly available materials, as it necessitates the respective components to have the requisite solubility properties as regards between themselves as well as with respect to the surfactant of the composition, which are generally difficult to achieve simultaneously.

Suitable oils for use in the invention include silicone-type oils which are capable of dissolving the cosmetic agent(s).

Particularly preferred are phenylsilicones, having for example viscosities in the range 10 to 10,000 centipoise (at 25°C).

Examples of suitable phenylsilicone oils include the phenyl-containing silicone fluids SF1023, SF1154 and SF1265 ex General Electric and phenyl methyl silicone oil DC550, phenyl trimethicone fluid DC556 and phenylmethyl polysiloxane fluid DC710 ex Dow Corning.

Certain alkyl-based silicone oils are also suitable, such as the dioctyl-modified cyclic silicone FZ3109 ex Wacker.

It is possible that other alkyl-silicones may be suitable, but these may generally be expected to be less useful, owing to their frequent inability to dissolve (sufficiently) the hydrophobic materials which are the preferred cosmetic agent(s).

Examples of such other silicone oils may include cyclic or linear alkylpolysiloxanes, arylpolysiloxanes and alkylarylpolysiloxanes having a viscosity in the range 0.65 - 100,000 centipoise at 25°C.

Cyclic alkylpolysiloxanes include cyclic polydimethylsiloxanes of the formula:

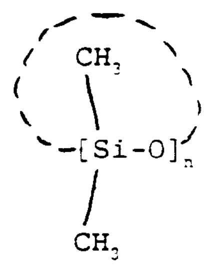

wherein n = 3 to 7, more preferably 4 to 5. Viscosities of these materials are generally less than 10 centipoise at 25°C.

Linear alkylpolysiloxanes will generally have viscosities of less than about 5 centipoise at 25°C and include linear polydimethylsiloxanes of the formula:

$$(CH_3)_3Si\text{-}O\text{-}[Si(CH_3)_2O]_nSi\ (CH_3)_3$$

wherein n = 1 to 7

Other preferred oil materials suitable for use in the invention include mineral oil and various organic oils.

Examples of organic oils include organic esters, especially isopropyl myristate and isopropyl palmitate, jojoba oil, fatty alcohols, fatty acids, fatty acid esters (in addition to those mentioned above), natural and synthetic oils and waxes, lanolin and derivatives thereof, oil sunscreens and sterols. Many other types and species of oils may also be suitable and generally any oil material or materials may be used, either singly or in combination, which satisfy the defined properties required of this invention.

## The emulsion

Another aspect of the present invention resides in the defined emulsion itself which has a continuous phase which comprises the one or more surfactants, at least as emulsifying agents, which may be present in an amount of from 0.1-50%, preferably 0.5-50%, more preferably 1 to 20%, especially 1-30%, for example 1-20%, typically 1-5% by weight of the emulsion.

Suitable emulsifiers are well-known in the art and include for example anionic surfactants such as alkylarylsulphonates e.g. sodium dodecylbenzene sulphonate, alkyl sulphates e.g. sodium lauryl sulphate, alkyl ether sulphates e.g. sodium lauryl ether sulphate nEO where n is from 1 to 20, alkylphenol ether sulphates e.g. octylphenol ether sulphate nEO where n is from 1 to 20, and sulphosuccinates e.g. sodium dioctylsulphosuccinate. Also suitable are nonionic surfactants such as alkylphenol ethoxylates e.g. nonylphenol ethoxylate nEO, where n is from 1 to 50, alcohol ethoxylates e.g. lauryl alcohol nEO, where n is from 1 or 50, ester ethoxylates e.g. polyoxyethylene monostearate where the number of oxyethylene units is from 1 to 30. Other types of emulsifier useful in the present invention, particularly other types of surfactant, are well described in the art literature.

If desired, the internal and/or continuous phases of the emulsion may contain one or more additional ingredients such as those normally found in cosmetic compositions.

If present, such additional ingredients will preferably be selected from the surfactants, conditioning agents, deposition polymers and other adjuncts as mentioned hereinbelow which may be used to formulate the final cosmetic cleansing compositions of the invention.

Such additional ingredients may, if necessary according to the amounts and characteristics of the materials forming the emulsion, include materials which alter the solubility of the cosmetic agent(s) in the oil(s) and/or modify the partition coefficient of the cosmetic agent(s) with respect to the internal oil phase and the continuous surfactant-comprising phase of the emulsion or final cosmetic composition into which the emulsion may be incorporated. Generally, however, good deposition of the cosmetic agent(s) is achieved without necessarily the use of such additional ingredients in the emulsion itself, a suitable amount of the cosmetic agent(s) preferentially remaining dissolved in the internal oil phase without partitioning to a significant degree into the micelles of surfactant in the continuous phase of the emulsion or, when the emulsion is incorporated into a final cosmetic composition, into the overall continuous phase of the cosmetic composition itself.

The continuous phase of the emulsion may, and preferably does, comprise water, preferably in an amount of from 0.01 to 99% by weight of the emulsion, more preferably from 0.1 to 70% by weight, even more preferably 0.5 to 50% by weight.

The cosmetic composition

Compositions in accordance with the present invention comprise the emulsion of internal oil phase having the surfactant-soluble cosmetic agent(s) preferably dissolved therein and surfactant-comprising continuous phase with or without further formulation ingredients.

Preferably, the emulsion constitutes from 0.01 to 100% by weight, more preferably from 1 to 70% by weight, even more preferably from 5 to 60% by weight of the final cosmetic composition.

It is most preferred that the emulsion is formulated with additional components to form the final cosmetic composition.

Additional components which may be formulated with the emulsion into the final composition include surfactants, conditioning agents, polyols, thickening agents, deposition aids, pearlescers, buffers, water as well as other optional adjunct materials such as antibacterial agents, antidandrutf agents such as zinc pyridinethione or Octopirox, foam boosters, perfumes, dyes, colouring agents, preservatives, viscosity modifiers (e.g. thickening agents such as sodium chloride, ammonium chloride) or various thickening polymers , proteins, polymers, moisturising agents, and herb extracts.

In preferred embodiments, the cosmetic compositions of the invention are in the form of shampoos for the hair or the body, comprising, in addition to the emulsion, at least one surfactant selected from anionic, cationic, nonionic, zwitterionic or amphoteric surfactants or mixtures thereof. This surfactant may only be present as an additional ingredient if sufficient for cleansing purposes is not provided as the emulsifier for the emulsion.

Suitable anionic surfactants are the alkyl sulphates, alkyl ether sulphates, alkaryl sulphonates alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauroyl isethionate and sodium N-lauryl sarcosinate. The most preferred anionic surfactants are sodium lauryl sulnhate, triethanolamine lauryl sulnhate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in compositions of the invention may include condensation products of aliphatic ($C_8$-$C_{18}$) primary or secondary linear or branched chain alcohols or phenols with alkylene oxides, usually ethylene oxide and generally having from 6 to 30 ethylene oxide groups.

Other suitable nonionics include mono- or di-alkyl alkanolamides, examples of which include coco mono- or di-ethanolamide and coco mono-isopropanolamide, and alkylpolyglycosides, examples of which are well known in the literature and commercially widely available.

Suitable cationic surfactants may include quaternary ammonium hydroxides, e.g. tetramethylammonium hydroxide, octyltrimethylammonium hydroxide, dodecyltrimethyammonium hydroxide, hexadecyltrimethylammonium hydroxide, cetyltrimethyl-ammonium hydroxide octyldimethylbenzylammonium hydroxide, decyldimethylbenzylammonium hydroxide, stearyldimethylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof e.g. chlorides, cetylpyridinium hydroxide and salts thereof eg chloride, Quaternium-5, Quaternium-31, Quaternium-18, and mixtures thereof.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

The surfactant(s) as an additional component may be present in the cosmetic composition in an amount of from 0.01 to 60% by weight of the total composition, more preferably from 0.1 to 50% by weight, even more preferably from 1 to 40% by weight.

Particularly when the cosmetic composition of the invention is in the form of a rinse-off cleansing and conditioning composition, e.g. for the hair, it preferably further comprises one or more conditioning agents.

Suitable conditioning agents include:

the cationic surfactants mentioned above; silicone resins, for example oligomerous alkylpolysiloxanes, arylpolysiloxanes or alkylarylpolysiloxanes composed of suitable combinations of $R_3SiO_{0.5}$ units, $R_2SiO$ units, $RSiO_{1.5}$ units and $SiO_2$ units, their ratio being selected so that the resin has an average formula of $R_nSiO_{[- n/2]}$ where R is $C_{1-6}$ alkyl or aryl and n is from 0.7 to 1.8; siloxane gums, such as high molecular weight alkylpolysiloxanes, arylpolysiloxanes or alkylarylpolysiloxanes having a viscosity greater than 100,000 est, preferably greater than 500,000, at 25°C; volatile silicones, such as low molecular weight cyclic or linear polydimethylsiloxanes; aminofunctional silicones, such as the amodimethicones and derivatives thereof; quaternary silicones; protein hydrolysates or quaternized protein hydrolysates; perfluoropolyether mateirals; cationic polymers, for example guar hydroxypropyltrimonium chloride, Quaternium -19, Quaternium -23, Quaternium -40, Quaternium -57, poly(dimethyldiallyammonium chloride), poly(dimethylbutenyl ammonium chloride)- , -bis(triethanolammonium chloride), poly(dipropyldiallylammonium chloride), poly(methyl-β-propaniodiallylammonium chloride), poly(diallylpiperidinium chloride), poly(vinyl pyridinium chloride), quaternised poly (vinyl alcohol), quaternised poly (dimethylaminoethylmethacrylate) and mixtures thereof.

The conditioning agent, if any, may be present in the cosmetic composition in a preferred amount of from 0.01 to 20% by weight of the total composition, more preferably 0.03 to 10%, especially 0.1 to 10%, typically 0.5 to 10% by weight.

Other ingredients to further enhance deposition of the cosmetic agent(s) from the cosmetic composition according to the invention may also be included therein. Examples of suitable deposition aids include cationic polymers, such as cationic derivatives of guar gum, and quaternary nitrogen-substituted cellulose ether derivatives.

Suitable cationic guar gum derivatives are those given the CTFA designation guar hydroxypropyl trimonium chloride, available commercially for example as JAGUAR C13S, which has a low degree of substitution of the cationic groups and a high viscosity. Other suitable materials include that known as JAGUAR C15, having a moderate degree of substitution and a low viscosity, JAGUAR C17 (high degree of substitution, high viscosity) and JAGUAR C16 which is a hydroxypropylated cationic guar derivative containing a low level of substituent groups as well as cationic quaternary ammonium groups. Also suitable is JAGUAR 162 which is a high transparency, medium viscosity guar having a low degree of substitution.

Suitable quaternary nitrogen-substituted cellulose ether derivatives include those available commercially as the Polymer JR series.

Such additional components used to formulate the final cosmetic compositions of the invention, if any, may be present in amounts such that the overall amounts of major components in the cosmetic composition are within the following preferred ranges (all amounts being % by weight of the total composition):
Surfactant: 5 to 40%, more preferably 7 to 35%, even more preferably 10 to 30%;
Conditioning agent, if any: 0.01 to 20%, more preferably 0.03 to 10%, especially 0.1 to 10%, typically 0.5 to 10%;
Deposition aid, if any: 0.01 to 10%, more preferably 0.02 to 5%, especially 0.05 to 1%;
Water, if any: 5 to 90%, more preferably 10 to 80%.

Other optional components, if any, may be present in conventional amounts, as will be appreciated by those skilled in the art.

## Preparation and final form of the cosmetic compositions

The cosmetic compositions in accordance with the invention may be prenared by first dissolving the cosmetic agent(s) in the oil material(s), together with any optional ingredients, and then emulsifying the solution thus obtained with the surfactant-comprising phase. Optional ingredients as described above may be added at the emulsification stage, the emulsification being effected by high speed stirring/mixing in accordance with conventional techniques.

If the emulsion is to be formulated into the final cosmetic composition with other ingredients, this is achieved by simple mixing, as is well-known in the art.

## Use of the cosmetic composition

The compositions of the invention may take any suitable form appropriate to the cosmetic agent(s) which they contain and are intended to deposit. By suitable selection of essential and non-essential ingredients and relative amounts thereof, the compositions of the invention may be in the form for example of hair shampoos and other rinse-off hair cleaning compositions, body shampoos, shower gels, facial washing compositions, bath foams and the like. Preferred compositions in the form of body or hair shampoos may be applied to the skin or hair (especially wet hair), as appropriate, and worked to create a lather. The lather may be retained at

the applied site for a short time, e.g. one or several minutes, before rinsing, or may be immediately rinsed. The procedure may be repeated if desired.

Retention of the lather at the site of application and repetition of the application régime may be of additional benefit in enhancing even further the amount or rate of deposition of the cosmetic agent(s) on the skin or hair surface.

The invention is further illustrated by the following examples, which are not intended to limit in any way the scope of the claimed invention.

Examples

Preparation of cosmetic compositions

Cosmetic compositions in accordance with the invention in the form of hair shampoos were prepared by first preparing emulsions having an internal phase of a solution of a sunscreen material (Parsol MCX) in an oil, and a continuous phase comprising surfactant, glycerol and water. The sunscreen was initially dissolved in the oil and the solution was then emulsified with the remaining components, with high speed stirring, to form the emulsion.

The compositions of the emulsions prepared are shown in Table I below. All amounts are expressed in % by weight of the emulsion, unless otherwise stated.

Table I

| | Emulsion | | |
|---|---|---|---|
| Ingredient | A | B | C |
| DC 556 | 23.5 | - | - |
| FZ 3109 | - | 23.5 | - |
| Mineral oil | - | - | 23.5 |
| Parsol MCX | 23.5 | 23.5 | 23.5 |
| Glycerol | 47 | 47 | 47 |
| Sodium lauryl ether sulphate 2EO | 1.5 | 1.5 | 1.5 |
| Water | 4.5 | 4.5 | 4.5 |

The above emulsions were formulated into shampoo compositions by mixing with a solution of SLES 2EO, JAGUAR C13S and Formalin to give shampoos having the compositions shown in Table II below. All amounts are expressed in % by weight of the total shampoo composition, unless otherwise stated.

Table II

| | Shampoo | | |
|---|---|---|---|
| Ingredient | A | B | C |
| D 556 | 2 | - | - |
| FZ 3109 | - | 2 | - |
| Mineral oil | - | - | 2 |
| Parsol MCX | 2 | 2 | 2 |
| Glycerol | 4 | 4 | 4 |
| SLES 2EO | 16 | 16 | 16 |
| Jaguar C13S | 0.3 | 0.3 | 0.3 |
| Formalin | 0.1 | 0.1 | 0.1 |
| Water | to 100 | to 100 | to 100 |

A reference shampoo composition was also prepared, by mixing the following ingredients, with stirring:

Table III

| Ingredient | % by weight |
|---|---|
| Parsol MCX | 2 |
| SLES 2EO | 16 |
| Jaguar C13S | 0.3 |
| Formalin | 0.1 |
| Water | to 100 |

The deposition of the cosmetic agent, Parsol MCX, from each of the shampoo compositions A, B, C and the reference shampoo was tested by the following procedure.

A dry hair switch (6" 3g Yugoslavian dark-brown) was placed into a polythene bag (2" x 8"). The bag was then filled with water and emptied, to dampen the switch. The shampoo ($0.5cm^3$) was applied evenly along the damp switch using a syringe. The switch was then massaged for 30 seconds to generate a lather and then left for 20 seconds. The bag was then filled with water and emptied six times, in order to rinse the switch. After removal from the bag, the switch was combed six times and dried for 30 minutes at 50-55°C. After drying, the switch was soaked in ethanol (100 $cm^3$) for 1 hour.

The absorbance of the ethanol solution in the region of 310nm was measured and recorded using an ultraviolet/visible spectrometer. From this data, the amount of Parsol MCX deposited on the switch was calculated.

The results for each shampoo tested are shown in Table IV below:

Table IV

| Shampoo | Concentration of Parsol MCX* (mg per gram of hair) |
|---|---|
| A | 0.460 (0.019) |
| B | 0.225 (0.009) |
| C | 0.304 (0.120) |
| Reference | 0.130 (0.044) |

*Average of three readings. Standard deviation shown in brackets.

It can be readily seen that the shampoo compositions in accordance with the present invention gave significantly increased levels of deposition of Parsol MCX, compared with the reference shampoo formed by simple mixing of ingredients.

In the same manner as for shampoos A to C above, 6 further shampoo compositions, D to I, were prepared by initially preparing the emulsions shown in Table V below and then formulating these as before into shampoos having the compositions shown in Table VI further below.

Table V

| Ingredient | Emulsion (wt%) | | | | | |
|---|---|---|---|---|---|---|
| | D | E | F | G | H | I |
| SF 1023 | 23.5 | - | - | - | - | - |
| SF 1154 | - | 23.5 | - | - | - | - |
| SF 1265 | - | - | 23.5 | - | - | - |
| DC 710 | - | - | - | 23.5 | - | - |
| DC 550 | - | - | - | - | 23.5 | - |
| Jojoba Oil | - | - | - | - | - | 23.5 |
| Parsol MCX | 23.5 | 23.5 | 23.5 | 23.5 | 23.5 | 23.5 |
| Glycerol | 47.0 | 47.0 | 47.0 | 47.0 | 47.0 | 47.0 |
| SLES 2EO | 1.5 | 1.5 | 1.5 | 1.5 | 1.5 | 1.5 |
| Water | to 100.0 | to 100.0 | to 100.0 | to 100.0 | to 100.0 | to100.0 |

Table VI

| Ingredient | Shampoo (wt%) | | | | | |
|---|---|---|---|---|---|---|
| | D | E | F | G | H | I |
| SF 1023 | 2.0 | - | - | - | - | - |
| SF 1154 | - | 2.0 | - | - | - | - |
| SF 1265 | - | - | 2.0 | - | - | - |
| DC 710 | - | - | - | 2.0 | - | - |
| DC 550 | - | - | - | - | 2.0 | - |
| Jojoba Oil | - | - | - | - | - | 2.0 |
| Parsol MCX | 2.0 | 2.0 | 2.0 | 2.0 | 2.0 | 2.0 |
| SLES 2EO | 16.0 | 16.0 | 16.0 | 16.0 | 16.0 | 16.0 |
| Jaguar C13S | 0.3 | 0.3 | 0.3 | 0.3 | 0.3 | 0.3 |
| Water | to 100.0 | to 100.0 | to 100.0 | to 100.0 | to 100.0 | to 100.0 |

Deposition of Parsol MCX from shampoos D to I was measured using the protocol described above. The results are given in Table VII below.

Table VII

| Shampoo | Concentration of Parsol MCX* (mg per gram of hair) |
|---|---|
| D | 0.364 (0.026) |
| E | 0.296 (0.012) |
| F | 0.336 (0.081) |
| G | 0.425 (0.053) |
| H | 0.467 (0.098) |
| I | 0.338 (0.137) |

*Average of five readings. Standard deviation shown in brackets.

The following shampoos J and K in accordance with the present invention were prepared by formulating emulsions E and F as above into shampoos having the compositions shown in Table VIII below. Also prepared was a comparative shampoo composition L not containing an internal oil phase for dissolving the surfactant-soluble sunscreen, which was prepared by direct mixing of the ingredients in accordance with the prior art.

Table VIII

| Ingredient | Shampoo (wt%) | | |
|---|---|---|---|
| | J | K | L |
| SF 1154 | 2.0 | - | - |
| SF 1265 | - | 2.0 | - |
| Parsol MCX | 2.0 | 2.0 | 2.0 |
| SLES 2EO | 16.0 | 16.0 | 16.0 |
| Tegobetaine C | 2.0 | 2.0 | 2.0 |
| Euperlan PK810 | 10.0 | 10.0 | 10.0 |
| Jaguar C13S | 0.3 | 0.3 | 0.3 |
| Potassium sorbate | 0.5 | 0.5 | 0.5 |
| Citric acid | 0.2 | 0.2 | 0.2 |
| Sodium chloride | 0.75 | 0.75 | 0.75 |
| Water | to 100 | to 100 | to 100 |

Using the same protocol as above, deposition of Parsol MCX from shampoo compositions J, K and L was measured and the results are given in Table IX below.

Table IX

| Shampoo | Concentration of Parsol MCX* (mg per gram of hair) |
|---|---|
| J | 0.870 (0.086) |
| K | 1.057 (0.074) |
| L | 0.469 (0.055) |

*Average of three readings. Standard deviation shown in brackets.

## Claims

1. A rinse-off cleansing composition including one or more surfactant-soluble cosmetic agents for deposition onto hair or skin, the composition comprising a stable emulsion having a continuous phase comprising one or more surfactants and an internal phase comprising one or more oil materials, wherein the internal oil phase contains the said one or more surfactant-soluble cosmetic agents.

2. A cleansing composition according to claim 1, wherein the cosmetic agent is a surfactant-soluble material selected from: sunscreen materials, after-sun treatment materials, occlusive oils, emollients, humectants, therapeutic and essential oils, perfumes, antiperspirants, moisturisers, colour cosmetic materials and mixtures thereof.

3. A cleansing composition according to claim 2, wherein the cosmetic agent is a surfactant-soluble sunscreen material.

4. A cleansing composition according to any one of claims 1 to 3, wherein the oil material is a solvent for the said one or more cosmetic agents.

5. A cleansing composition according to claim 4, wherein the oil material is substantially surfactant-insoluble.

6. A cleansing composition according to claim 4 or claim 5, wherein the oil material is a silicone oil.

7. A cleansing composition according to claim 6, wherein the silicone oil is selection from phenylsilicones.

8. A cleansing composition according to claim 4 or claim 5, wherein the oil material is selected from mineral oil and organic oils.

9. A cleansing composition according to any preceding claim, which is in the form of a hair or body shampoo and contains surfactant in a total amount of from 5 to 40% by weight of the composition.

10. A cleansing composition according to any preceding claim, further comprising a conditioning agent.

11. A cleansing composition according to any preceding claim, further comprising a deposition aid for the one or more cosmetic agents.

12. A cleansing composition according to claim 11, wherein the deposition aid is selected from cationic derivatives of guar gum and quaternary nitrogen-substituted cellulose ether derivatives.

13. A process for preparing a rinse-off cleansing composition including one or more surfactant-soluble cosmetic agents for deposition onto hair or skin, the process comprising:
(i) dissolving the one or more cosmetic agents in one or more oil materials;
(ii) emulsifying the resulting solution with a phase comprising one or more surfactants to form a stable emulsion having a continuous phase comprising said one or more surfactants and an internal phase comprising said solution of one or more cosmetic agents in said one or more oil materials; and
(iii) optionally adding to the emulsion one or more additional ingredients for forming the said rinse-off cleansing composition.

14. A stable emulsion for use in the preparation of a rinse-off cleansing composition including one or more surfactant-soluble cosmetic agents for deposition onto hair or skin, the emulsion comprising a continuous phase comprising one or more surfactants and an internal phase comprising one or more oil materials, wherein the internal oil phase contains the said one or more surfactant-soluble cosmetic agents.

15. An emulsion according to claim 14, wherein the continuous phase comprises one or more surfactants in an amount of from 0.1 to 50% by weight of the emulsion.

16. An emulsion according to claim 14 or claim 15, wherein the continuous phase comprises water in an amount of from 0.01 to 99% by weight of the emulsion.

17. A method of facilitating and/or enhancing deposition of one or more surfactant-soluble cosmetic agents from a rinse-off cleansing composition, the method comprising providing the said one or more cosmetic agents in an internal oil phase of a stable emulsion, the emulsion having a continuous phase comprising one or more surfactants and an internal phase comprising one or more oil materials in which is contained the said one or more cosmetic agents.

18. Use of one or more oil materials for enhancing deposition of one or more surfactant-soluble cosmetic agents from a rinse-off cleansing composition onto hair or skin, wherein the said one or more cosmetic agents are contained in said one or more oil materials which form an internal phase of a stable emulsion, the emulsion having a continuous phase comprising one or more surfactants.